# EUROPÄISCHE PATENTSCHRIFT

(11) **EP 3 222 406 B1**
(45) Veröffentlichungstag und Bekanntmachung des Hinweises auf die Patenterteilung: **12.09.2018**
(21) Anmeldenummer: 16161715.4
(22) Anmeldetag: 22.03.2016
(51) Int. Cl.: B29C 55/06, B29C 55/18, B29C 55/00, B32B 27/20, B32B 27/08, B29C 55/02, B29C 44/56, B29C 71/02, B29K 23/00, B29K 105/04, B29K 105/16

(54) **VERFAHREN ZUR HERSTELLUNG EINER GEFÜLLTEN FOLIENBAHN**
METHOD FOR PRODUCING A FILLED FILM WEB
PROCÉDÉ DE FABRICATION D'UN FILM REMPLI

(43) Veröffentlichungstag der Anmeldung: 27.09.2017
(73) Patentinhaber: RKW SE, 67227 Frankenthal (DE)
(72) Erfinder: BÖRMANN, LUDWIG, 83547 Babensham (DE)
(74) Vertreter: Lang, Johannes

(56) Entgegenhaltungen:
- EP-A1- 2 565 013
- EP-A2- 0 768 168
- WO-A1-99/22930

## Beschreibung

Die Erfindung betrifft ein Verfahren zur Herstellung einer gefüllten Folienbahn, eine damit hergestellte Folienbahn sowie ihre Verwendung, beispielsweise im Hygienesektor.

Im Zuge der Umweltdiskussion zur Ressourcenschonung und Nachhaltigkeit wird es auf dem Gebiet der Folien, vor allem von Folien für Wegwerfprodukte aus dem Hygienesektor, von immer größerer Bedeutung, noch dünnere Folien als bisher herzustellen, um Rohstoffe einzusparen. Andererseits nimmt das Interesse an gefüllten und atmungsaktiven Folien zu, weil diese eine weitere Einsparung an Kunststoffen ermöglichen.

Aus der EP-A-0 768 168 und der EP-A-1 716 830 sind Verfahren zur Herstellung von im Hygienebereich einsetzbaren, ungefüllten Folien bekannt. An solche Hygienefolien werden aufgrund ihres Einsatzbereichs mehrere Anforderungen gestellt. Sie sollen flüssigkeitsdicht sein und über bestimmte haptische Eigenschaften verfügen, wie Weichheit, Geschmeidigkeit, raschelarmes Verhalten und textiler Griff. Folien im Hygienebereich sollen einen weichen, stoffähnlichen Griff besitzen. Insbesondere bei ihrer Verwendung für Inkontinenzprodukte soll die Geräuschentwicklung möglichst gering sein, d.h. die Folien sollen raschelarm sein. In Verbindung mit einem geringen Glanzgrad ergibt dies eine sehr textile Folie, was im Hygienebereich erwünscht ist. Hinzu kommt, dass in den letzten Jahren die in Windeln und Inkontinenzprodukten enthaltenen Saugkörper immer dünner geworden sind, was insbesondere durch die Verwendung von Superabsorber-Polymeren ermöglicht wurde. Diese Superabsorber-Polymere werden in Form grobkörniger Pulver eingesetzt, und die Hygienefolien müssen eine derartige Festigkeit besitzen, dass ein Durchstoßen der Folie durch die einzelnen Körner, z.B. bei Belastung durch Hinsetzen oder sonstige Bewegung des Trägers, mit hoher Sicherheit vermieden wird. Eine Bildung von Löchern (*"Pinholes"*) durch Superabsorber-Polymere und ein Platzen der Fertigfolienprodukte in den Verpackungseinheiten müssen vermieden werden. Eine weitere Anforderung an Hygienefolien besteht in einer Mindestzugfestigkeit, die für eine Verarbeitung der Folienbahnen auf den schnell laufenden Maschinen (Konverter) der Hersteller von z.B. Windeln und Damenbinden erforderlich ist. Diese Mindestzugfestigkeit wird in der Regel für 5%, 10 % oder 25 % Dehnung in Maschinenrichtung (MD) oder Querrichtung (CD) angegeben. Derzeit soll die Zugfestigkeit bei 5 % Dehnung (5%-Modul) in Maschinenrichtung wenigstens 2,5 N/inch betragen. Darüber hinaus sollen Folien für Hygieneanwendungen bestimmte Festigkeiten aufweisen, beispielsweise einlagige Backsheets eine Längsreißfestigkeit von wenigstens 10 N/inch und eine Querreißfestigkeit von wenigstens 5 N/inch. Wird das Backsheet mit einem Vlies laminiert, soll die Längsreißfestigkeit wenigstens 5 N/inch und die Querreißfestigkeit wenigstens 2 N/inch betragen.

Verfahren zur Herstellung atmungsaktiver Folien sind beispielsweise aus der EP 0 921 943 B1, EP 1 226 013 B1, EP 1 711 330 B1 und GB 2 364 512 B bekannt. Atmungsaktive Folien müssen den obigen Anforderungen an mechanische Eigenschaften wie ungefüllte Folien genügen und weiterhin flüssigkeitsdicht sein. Auch bei ihnen werden geringe Dicken angestrebt. Dadurch können sich mehrere Probleme ergeben.

Zur Herstellung von atmungsaktiven Folien werden Folien mit etwa 60 % inertem Material gefüllt und nach dem Extrudieren einem Reckprozess (meist einem Recken in Maschinenrichtung) unterzogen, um die Folie atmungsaktiv zu machen. Als Füllstoff wird üblicherweise Kreide (CaCO₃) in einer Partikelgröße von 0,8-2µm eingesetzt. Beim Reckprozess werden die elastischen polymeren Anteile der Folie gedehnt und es entstehen Poren am Rand der Kreidekörner hin zur Polymermatrix. Durch Streuungen der Kreidepartikelgrößen (bis 12 µm und größer) entstehen auch immer Porengrößen, die zu Dichtigkeitsproblemen führen können. Dieses Problem wird verstärkt, wenn zur Herstellung möglichst dünner, atmungsaktiver Folien beim Recken relativ hohe Reckgrade von z.B. 2:1 bis 3:1 nötig sind. Teilweise zeigen in Maschinenrichtung gereckte Folien auch geringe Sicherheit gegen Undichtigkeiten (sogenanntes Leakage). Es besteht auch die Gefahr, dass an einigen Stellen in der Folie die erzeugten Poren zu groß werden (>1µm) und damit ein Problem der Durchnässung entsteht (d.h. Flüssigkeitsdurchstoßfestigkeitswerte (Liquid Impact Werte) größer als 3 g/m² vorliegen). Erwünscht sind Werte für die Flüssigkeitsdurchstoßfestigkeit von kleiner 2 g/m² oder gar kleiner 1,5 g/m².

Bekanntlich haben Folien einen sogenannten Rückerinnerungseffekt. Das bedeutet, dass Folien, die beispielsweise bei 80°C gereckt und bei 100°C anschließend einem Annealing unterzogen wurden, bei nochmaligen Erreichen dieser Temperaturen, z. B. bei sehr heißen Hotmeltklebstoffen (ca. 160°C) im Konverter, zu schrumpfen versuchen. Dieses Problem tritt gerade bei kreidegefüllten Folien wegen ihrer guten Wärmeleitfähigkeit und bei besonders dünnen Folien auf. Bei zu hoher Temperaturbelastung bzw. zu geringer Foliendicke können dann sehr schnell unerwünschte Löcher (sogenannter Burn-Through-Effekt) entstehen.

Üblicherweise werden heutzutage atmungsaktive Folien nach dem Reckprozess für einige Tage zwischengelagert und die Nachkristallisation abgewartet, bevor es zur Weiterverarbeitung, z.B. Bedrucken, kommt, da die Folien nachschrumpfen können. Soll also eine Folie bedruckt werden, muss nach dem Reckvorgang und vor dem Druckvorgang eine Kristallisationszeit von etwa 1 bis 3 Tagen abgewartet werden. Dieser Vorgang verursacht sehr hohe Kosten und behindert ein Inline-Drucken der Folien.

Gefüllte und gereckte Folien neigen auf den fertigen Rollen zum Blocking. Blocking bedeutet, dass die Folienlagen durch Nachschrumpf derart aneinander haften, dass beim Abrollen Schwierigkeiten auftreten, z.B. dass die Folie sogenannte Spiralrisse zeigt. Bei Spiralrissen haftet die Folie partiell auf der darunterliegenden Folienlage. Dies führt zu Abrissen der Folie beim Abrollvorgang, wovon die Bereiche in der Nähe des Schnittspiegels besonders betroffen sind. Blocking stellt vor allem beim Abrollen von dünnen Folien ein Problem dar.

In Maschinenrichtung (MD) gereckte Folien haben eine geringe Durchstoßfestigkeit gegenüber scharfkantigen Superabsorber-Körnern, die häufig in Hygieneprodukten zur Flüssigkeitsaufnahme verwendet werden. Da diese Körner oft in direktem Kontakt mit der Folie stehen, kann es zu Pinholes und Undichtigkeiten (Leakage) im Fertigprodukt kommen. Zudem zeigen gefüllte und MD-gereckte Folien eine geringe MD-Weiterreißfestigkeit und eine geringe MD-Einreißfestigkeit. Geringste Verletzungen auf der Rollenstirnseite oder ein geringes Blocking der Folie auf der Rolle können zum Einreißen und Weiterreißen führen, wodurch sich Spiralrisse bilden.

Der Reckprozess verstärkt generell die Unterschiede von Dick- und Dünnstellen in der Folie und kann zusätzlich zur Randverdickung führen, auch "Neck In" genannt. Beide Effekte verursachen auf den fertigen Rollen sogenannte Kolbenringe. Das bedeutet, dass beim Abrollen dieser Rollen lange Kanten oder Durchhänger in der Folie entstehen, die wiederum zu großen Schwierigkeiten beim Konvertierprozess führen können (z.B. CD-Versatz der Folie). Hohe Reckgrade verstärken die Randverdickung (Neck In) der Folie, den Nachschrumpf der Folie nach dem Reckprozess und eine sehr geringe Weitereißfestigkeit der Folie in Maschinenrichtung. Oft werden die Rollen auch in sogenannten Mutterrollen zwischengelagert und erst nach erfolgtem Nachschrumpf (Kristallisation) einem Schneidumroller zugeführt, in dem sie dann auf gewünschte Kundenbreite zugeschnitten werden. Der Nachschrumpf der atmungsaktiven Folien kann auf den fertigen Rollen eine erhebliche Lagenpressung auslösen, welche wiederum zwischen den Folienlagen ein Blocking auslösen und zu Spiralrissen beim Abrollen der Folie führen kann.

Insbesondere bei Backsheets (Unterlagenschichten für Windeln und Hygieneprodukte) lösen Randverdickungseffekte, wie Durchhänger und lange Folienkanten, große Probleme beim Einlaufen in die Konverter aus, da zum einen der Reckprozess in Maschinenrichtung sehr stark vorhandene Dick- und Dünnstellen verstärkt und zum anderen ein Versatz der Folien in Querrichtung (CD) eintreten kann, was letztendlich zu einem Stillstand des Konverters führen kann. Daher ist ein planliegendes Einlaufen von Backsheets in Konvertern von großer Bedeutung.

EP 2 565 013 A1 offenbart ein Verfahren zum Recken einer Ausgangsfolienbahn aus thermoplastischem Polymermaterial, wobei die Ausgangsfolienbahn bis zum teilweise schmelzflüssigen Zustand erwärmt und anschließend in einem gekühlten Walzenspalt abgekühlt wird.

Zur Lösung einer oder mehrerer dieser Probleme schlägt die vorliegende Erfindung vor, eine gefüllte, mikroporöse Ausgangsfolienbahn bis zum teilweise schmelzflüssigen Zustand zu erwärmen und anschließend in einem gekühlten Walzenspalt schnell abzukühlen. Die gefüllte mikroporöse Ausgangsfolienbahn enthält Mikroporen. Bestehen zwischen den Poren Verbindungen, ist die Folie atmungsaktiv. Überraschenderweise wurde gefunden, dass die Poren bei gefüllten atmungsaktiven und gefüllten nicht-atmungsaktiven Folien sich beim Erwärmen der Folie in den teilweise schmelzflüssigen Zustand nicht verschließen, sondern bestehen bleiben. Durch den teilweise schmelzflüssigen Zustand und das anschließende Abkühlen der Folie werden die Eigenschaften der Folie signifikant verbessert und damit die obengenannten Probleme gelöst.

Somit betrifft die Erfindung ein Verfahren zur Herstellung einer gefüllten Folienbahn aus einer mikroporösen Ausgangsfolienbahn aus thermoplastischem Polymermaterial, die wenigstens eine niedrig schmelzende Polymerkomponente, eine hoch schmelzende Polymerkomponente und einen Füllstoff enthält, wobei das Verfahren folgende Schritte umfasst: Erwärmen der mikroporösen Ausgangsfolienbahn bis zum teilweise geschmolzenen Zustand, bei dem sich wenigstens eine niedrig schmelzende Polymerkomponente im schmelzeflüssigen Zustand und wenigstens eine hoch schmelzende Polymerkomponente nicht im schmelzeflüssigen Zustand befindet, und Abkühlen durch Führen der teilweise geschmolzenen Folienbahn durch einen gekühlten Walzenspalt.

Die gefüllte mikroporöse Ausgangsfolienbahn kann atmungsaktiv oder nichtatmungsaktiv sein. Der Begriff "mikroporös" bedeutet vorliegend, dass im wesentlichen Poren einer Größe von 0,1 bis 5 µm vorliegen. Im wesentlichen bedeutet, dass wenigstens 90 % der Poren, bevorzugt 95 %, stärker bevorzugt 99% der Poren, oder gar 99,9% der Poren eine Größe 0,1 bis 5 µm haben und die restlichen Poren etwas größer sind, im allgemeinen bis zu 15 µm.

Die mikroporöse Ausgangsfolienbahn kann mehrlagig oder mehrschichtig sein. In bevorzugten Ausführungsformen ist die mikroporöse Ausgangsfolienbahn eine coextrudierte Folienbahn. In anderen Ausführungsformen ist die mikroporöse Ausgangsfolienbahn nicht coextrudiert. In weiteren Ausführungsformen ist die mikroporöse Ausgangsfolienbahn eine mehrlagige oder mehrschichtige, nicht coextrudierte Folienbahn.

In anderen Ausführungsformen ist die mikroporöse Ausgangsfolienbahn einlagig.

In einer bevorzugten Ausführungsform der Erfindung ist die Ausgangsfolienbahn in Maschinenrichtung (MD) oder in Querrichtung (CD), oder in Maschinen- und Querrichtung gereckt. In einer anderen bevorzugten Ausführungsform ist die Ausgangsfolienbahn bei der Herstellung, beispielsweise nach ihrer Extrusion, bei einem Reckverhältnis von 1,2:1 bis 4:1, insbesondere bei 1,3:1 bis 3,5:1 oder 1,5:1 bis 3:1 gereckt worden.

Vorzugsweise enthält die mikroporöse Ausgangsfolienbahn 10 bis 90 Gew.-% Füllstoff, insbesondere 20 bis 80 Gew.-% Füllstoff, bevorzugt 30 bis 75 Gew.-% Füllstoff, stärker bevorzugt 50 bis 60 Gew.-% Füllstoff, bezogen auf 100 Gew.-% der Ausgangsfolienbahn. In einer bevorzugten Ausführungsform ist die Ausgangsfolienbahn atmungsaktiv. In einer anderen bevorzugten Ausführungsform ist die mikroporöse Ausgangsfolienbahn nicht atmungsaktiv.

Die mikroporöse Ausgangsfolienbahn weist eine Wasserdampfdurchlässigkeit im Bereich von 500 bis 5000 g/m² in 24h bei Messung gemäß ASTM E398 bei 37,8 °C und 90 % relativer Feuchtigkeit auf. Hierin angegebene Werte oder Wertebereiche für die Wasserdampfdurchlässigkeit beziehen sich auf dieses Messverfahren. Beispielsweise kann mit einem Messgerät Lyssy gemessen werden.

In weiterhin bevorzugten Ausführungsformen wird eine Ausgangsfolienbahn mit 15 bis 85 Gew.-% niedrig schmelzender Polymerkomponente und 85 bis 15 Gew.-% hoch schmelzender Polymerkomponente, bezogen auf 100 Gew.-% niedrig und hoch schmelzende Polymerkomponente, verwendet. Bevorzugt wird eine Ausgangsfolienbahn mit wenigstens einem Polyethylen als niedrig schmelzender Polymerkomponente und mit wenigstens einem Polypropylen als hoch schmelzender Polymerkomponente verwendet.

Insbesondere erfolgt die Erwärmung der Ausgangsfolienbahn auf 5 bis 20 °C unterhalb des Kristallitschmelzpunktes wenigstens einer hoch schmelzenden Polymerkomponente. Bevorzugt erfolgt die Erwärmung der Ausgangsfolienbahn über einen Heizzylinder, wobei die Temperatur des Heizzylinders so gewählt wird, dass über die Umschlingungsstrecke des Heizzylinders die Erwärmung durchgeführt wird.

In bevorzugten Ausführungsformen erfolgt die Erwärmung der mikroporösen Ausgangsfolienbahn über einen Heizzylinder, wobei zwischen dem Heizzylinder und der Folienbahn kein Vlies vorliegt. In diesen Ausführungsformen steht die Folienbahn in direktem Kontakt mit dem Heizzylinder. Möglich sind auch Ausführungsformen, in denen zwischen dem Heizzylinder und der Folienbahn kein Vlies vorliegt und eine Vliesbahn auf der Folienbahn aufliegt oder darüber geführt wird. In anderen bevorzugten Ausführungsformen wird die Erwärmung der mikroporösen Ausgangsfolienbahn über einen Heizzylinder in Abwesenheit eines Vlieses durchgeführt. Das bedeutet, es liegt kein Vlies zwischen dem Heizzylinder und der Folienbahn vor und es liegt kein Vlies auf der Folienbahn auf. In anderen Ausführungsformen mit Vlies liegt ein Vlies zwischen dem Heizzylinder und der Folienbahn vor.

In weiterhin bevorzugten Ausführungsformen wird die Folienbahn im gekühlten Walzenspalt einer Abkühlung auf wenigstens 10 bis 30 °C unterhalb des Kristallitschmelzpunktes wenigstens einer niedrig schmelzenden Polymerkomponente unterworfen. Vorzugsweise wird der gekühlte Walzenspalt durch eine Prägewalze und eine Gummiwalze gebildet. Die Folienbahn kann nach dem Abkühlen bedruckt werden.

Außerdem betrifft die Erfindung die mit den beschriebenen Verfahren hergestellten Folienbahnen aus einer Ausgangsfolienbahn mit einer Wasserdampfdurchlässigkeit im Bereich von 500 bis 5000 g/m² in 24h bei Messung gemäß ASTM E398 bei 37,8 °C und 90 % relativer Feuchtigkeit, beispielsweise Folienbahnen mit einem Flächengewicht von 1 bis 30 g/m², insbesondere von 5 bis 25 g/m² , bevorzugt von 7 bis 20 g/m², stärker bevorzugt von 10 bis 20 g/m², sowie deren Verwendung, insbesondere im Hygiene- oder Medikalbereich, zum Beispiel für Backsheets in Windeln, für Betteinlagen oder Damenbinden. Daneben betrifft die Erfindung die Verwendung der hergestellten Folienbahnen im Baubereich, z. B. als Abdeckfolien, oder als Automobilschutzfolien.

Bevorzugte Ausführungsformen der Erfindung sind in der nachfolgenden Beschreibung, den Figuren, dem Beispiel und den Unteransprüchen beschrieben.

Die Figuren zeigen:
Figur 1 zeigt eine bevorzugte Ausführungsform zur Durchführung des erfindungsgemäßen Verfahrens.
Figur 2 zeigt den Schrumpf und das Flächengewicht von Folien in Abhängigkeit von ihrer Heizzylindertemperatur.
Figur 3 zeigt den Schrumpf für eine Folie und eine Vergleichsfolie in Abhängigkeit von der Wasserbadtemperatur.

In der vorliegenden Erfindung beziehen sich die angegebenen Schmelzpunkte, Schmelzbereiche und Kristallitschmelzpunkte auf eine Bestimmung gemäß DSC (Differential Scanning Calorimetry).

Erfindungsgemäß enthält oder umfasst die Ausgangsfolienbahn wenigstens eine niedrig schmelzende Polymerkomponente und wenigstens eine hoch schmelzende Polymerkomponente. Mit anderen Worten, die Ausgangsfolienbahn enthält eine oder mehrere niedrig schmelzende Polymerkomponente(n) und eine oder mehrere hoch schmelzende Polymerkomponente(n). Das Gleiche bedeuten die in der vorliegenden Erfindung weiterhin verwendeten Begriffe "eine niedrig schmelzende Polymerkomponente" und "eine hoch schmelzende Polymerkomponente", d.h. auch sie umfassen eine oder mehrere niedrig bzw. hoch schmelzende(n) Polymerkomponente(n). Vorzugsweise enthält die Ausgangsfolienbahn eine, bevorzugt zwei, niedrig schmelzende Polymerkomponente(n). Bevorzugt enthält sie eine, insbesondere zwei, hoch schmelzende Polymerkomponente(n). In anderen Ausführungsformen der Erfindung enthält sie bevorzugt drei niedrig schmelzende Polymerkomponenten und/oder drei hoch schmelzende Polymerkomponenten. Ob ein Polymermaterial der Ausgangsfolienbahn zur niedrig schmelzenden Polymerkomponente oder zur hoch schmelzenden Polymerkomponente gehört, bestimmt sich erfindungsgemäß nach dem jeweiligen Kristallitschmelzpunkt, Schmelzpunkt oder Schmelzbereich des Polymermaterials im Verhältnis zur Erwärmungstemperatur. Bei gegebener Erwärmungstemperatur werden die schmelzeflüssigen Polymermaterialien der niedrig schmelzenden Polymerkomponente und die nicht-schmelzeflüssigen Polymermaterialien der hoch schmelzenden Polymerkomponente zugeordnet.

Bekanntlich besitzen Polymere keinen scharf definierten Schmelzpunkt, sondern einen Schmelzbereich, wobei sich jedoch den kristallinen Bereichen eines Polymeren ein Kristallitschmelzpunkt zuordnen lässt. Dieser Kristallitschmelzpunkt liegt stets höher als der Schmelzpunkt oder Schmelzbereich der nicht-kristallinen Bestandteile. Der schmelzeflüssige Zustand ist dadurch beschrieben, dass der Schubmodul gegen Null geht. Im Falle von Polymeren mit kristallinen Bereichen sind letztere dann nicht mehr nachweisbar. Der Schubmodul kann beispielsweise nach ISO 6721-1 & 2 bestimmt werden. Bei der vorliegenden Erfindung wird die Ausgangsfolienbahn auf eine Temperatur erwärmt, bei der für die niedrig schmelzende Polymerkomponente der Schubmodul Null beträgt und für die hoch schmelzende Polymerkomponente der Schubmodul nicht Null ist. Bei der niedrig schmelzenden Polymerkomponente sind dann keine kristallinen Bereiche mehr nachweisbar und die niedrig schmelzende Polymerkomponente befindet sich im schmelzeflüssigen Zustand. Hingegen sind bei der hoch schmelzenden Polymerkomponente noch kristalline Bereiche nachweisbar und diese befindet sich unterhalb des schmelzeflüssigen Zustands. In Summe ist der Schubmodul des gesamten Polymermaterials der Ausgangsfolienbahn somit nicht Null und kristalline Bereiche der hoch schmelzenden Polymerkomponente sind noch nachweisbar. Es liegt also eine teilweise aufgeschmolzene Folienbahn vor.

Als Materialien für die beiden Polymerkomponenten der Ausgangsfolienbahn kommen im Prinzip alle thermoplastischen Polymere in Betracht, die über entsprechende Schmelzpunkte verfügen. Hierzu sind zahlreiche Handelsprodukte auf dem Markt erhältlich. Vorzugsweise werden verschiedene Polyolefine, insbesondere Polyethylene, Polypropylene, Copolymerisate von Ethylen und Propylen, Copolymerisate von Ethylen und Propylen mit anderen Comonomeren, oder Gemische davon eingesetzt. Weiterhin sind Ethylenvinylacetat (EVA), Ethylenacrylat (EA), Ethylenethylacrylat (EEA), Ethylenacrylsäure (EAA), Ethylenmethylacrylat (EMA), Ethylenbutylacrylat (EBA), Polyester (PET), Polyamid (PA), z.B. Nylon, Ethylenvinylalkohole (EVOH), Polystyrol (PS), Polyurethan (PU), thermoplastische Olefinelastomere oder thermoplastische Ether-Ester-Blockelastomere (TPE-E) geeignet.

Die Gesamtmenge an niedrig schmelzender Polymerkomponente beträgt vorzugsweise 90 bis 10 Gew.-%, insbesondere 90 bis 20 Gew.-%, bevorzugt 80 bis ₃₀ Gew.-%, stärker bevorzugt 80 bis 40 Gew.-%, am meisten bevorzugt 70 bis 50 Gew.-%. Die Gesamtmenge an hoch schmelzender Polymerkomponente beträgt vorzugsweise 10 bis 90 Gew.-%, insbesondere 10 bis 80 Gew.-%, bevorzugt 20 bis 70 Gew.-%, stärker bevorzugt 20 bis 60 Gew.-%, am meisten bevorzugt 30 bis 50 Gew.-%, jeweils bezogen auf 100 Gew.-% niedrig schmelzende und hoch schmelzende Polymerkomponente. Alternativ beträgt vorzugsweise die Gesamtmenge an niedrig schmelzender Polymerkomponente 85 bis 15 Gew.-%, insbesondere 75 bis 25 Gew.-%, und die Gesamtmenge an hoch schmelzender Polymerkomponente 15 bis 85 Gew.-%, insbesondere 25 bis 75 Gew.-%, wiederum bezogen auf 100 Gew.-% niedrig und hoch schmelzende Komponente. Bei diesen Mengenangaben kann es sich beispielsweise bei der niedrig schmelzenden Polymerkomponente um ein oder mehrere Polyethylen(e) und bei der hoch schmelzenden Polymerkomponente um ein oder mehrere Polypropylen(e) handeln.

In einer besonders bevorzugten Ausführungsform enthält die Ausgangsfolienbahn wenigstens ein Polyethylen als niedrig schmelzende Polymerkomponente und wenigstens ein Polypropylen als hoch schmelzende Polymerkomponente.

Vorzugsweise enthält die niedrig schmelzende Polymerkomponente Ethylenpolymere oder besteht aus diesen, wobei sowohl Ethylenhomopolymere als auch Ethylencopolymere mit Ethylen als Hauptmonomerem sowie Gemische (Blends) von Ethylenhomopolymeren und Ethylencopolymeren geeignet sind. Geeignete Ethylenhomopolymere sind LDPE (Low Density Polyethylene), LLDPE (Linear Low Density Polyethylene), MDPE (Medium Density Polyethylene) und HDPE (High Density Polyethylene). Bevorzugte Comonomere für Ethylencopolymere sind andere Olefine als Ethylen mit Ausnahme von Propylen, z. B. Buten, Hexen oder Octen. Vorzugsweise liegt bei den Ethylencopolymeren der Comonomergehalt unter 20 Gew.-%, insbesondere unter 15 Gew.-%. In einer bevorzugten Ausführungsform besteht die niedrig schmelzende Polymerkomponente ausschließlich aus einem Ethylenhomopolymer oder Gemischen von Ethylenhomopolymeren, z. B. aus LDPE und LLDPE, die jeweils in Mengen von 10 bis 90 Gew.-% enthalten sein können, sowie 0 bis 50 Gew.-% MDPE. Spezielle Beispiele sind ein Polyethylen aus 60 Gew.-% LDPE und 40 Gew.-% LLDPE oder ein Polyethylen aus 80 Gew.-% LDPE und 20 Gew.-% LLDPE.

Neben den Ethylenhomopolymeren und/oder Ethylencopolymeren kann die niedrig schmelzende Polymerkomponente auch andere thermoplastische Polymere enthalten. Diese thermoplastischen Polymeren sind nicht beschränkt, solange hierdurch die Temperatur, bei der sich die gesamte niedrig schmelzende Polymerkomponente im schmelzeflüssigen Zustand befindet, nicht zu nahe an die Temperatur heranrückt, bei der sich die hoch schmelzende Polymerkomponente im schmelzeflüssigen Zustand befindet. Es ist auch möglich, dass die niedrig schmelzende Polymerkomponente ein Polypropylen enthält, dessen Schmelzpunkt oder Schmelzbereich nicht höher als der eines Ethylenhomopolymeren oder Ethylencopolymeren liegt oder aber zwar höher als diese, jedoch immer noch tiefer als die eingesetzte Erwärmungstemperatur. Bekanntlich gibt es hochkristallines isotaktisches, weniger kristallines syndiotaktisches und amorphes ataktisches Polypropylen, die über unterschiedliche Schmelzpunkte, Schmelzbereiche oder Kristallitschmelzpunkte verfügen. Bei Verwendung von amorphem ataktischem Polypropylen, das einen wesentlich niedrigeren Schmelzpunkt oder Schmelzbereich als isotaktisches und gegebenenfalls auch syndiotaktisches Polypropylen hat, wäre dieses in Abhängigkeit von der Erwärmungstemperatur gegebenenfalls der niedrig schmelzenden Polymerkomponente zuzurechnen.

Vorzugsweise enthält die hoch schmelzende Polymerkomponente wenigstens ein Polypropylen, dessen Schmelzpunkt, Schmelzbereich oder Kristallitschmelzpunkt wesentlich höher als der der niedrig schmelzenden Polymerkomponente liegt. Geeignetes Polypropylen ist insbesondere isotaktisches Polypropylen. Es kann auch syndiotaktisches Polypropylen verwendet werden, sofern dessen Schmelzpunkt, Schmelzbereich oder Kristallitschmelzpunkt wesentlich höher als der der niedrig schmelzenden Polymerkomponente liegt. Geeignete Polypropylene sind im Handel erhältlich, beispielsweise für die Herstellung von Blasfolien und/oder Castfolien (Gießfolien).

Die hoch schmelzende Polymerkomponente kann sowohl Propylenhomopolymere als auch Propylencopolymere mit Propylen als Hauptmonomerem umfassen. Bei den Propylencopolymeren ist hierbei der Anteil des Comonomeren, d. h. das Nicht-Propylen, in Anhängigkeit von den anderen Komponenten und der Erwärmungstemperatur der niedrig oder hoch schmelzenden Polymerkomponente zuzurechnen. Geeignete Comonomere für Propylencopolymere sind andere Olefine als Propylen, vorzugsweise Ethylen. Bei den Propylen-Ethylen-Copolymeren beträgt der Ethylenanteil vorzugsweise 2 bis 30 Gew.-%, besonders bevorzugt 2 bis 20 Gew.-% und insbesondere 2 bis 15 Gew.-%, wobei in der Praxis bei einem Ethylengehalt von 3 bis 20 Gew.-% sehr gute Ergebnisse erhalten werden. Diese Zahlenwerte gelten auch für die anderen Olefine.

Nachfolgend sind für einige Polyethylene und Polypropylene die Schmelzbereiche angegeben:
LDPE: 110 - 114°C;
LLDPE: 115 - 130°C;
HDPE: 125 -135 °C;
Propylen-Homopolymere: 150 - 165°C;
Propylen-Ethylen-Copolymere: 120 - 162°C, bei sehr wenig Ethylen auch höhere Temperaturen möglich;
Bimodale Propylen-Ethylen-(Homo)Copolymere: 110 -165 °C.

Es ist auch möglich, sogenannte bimodale Polypropylene zu verwenden. Dabei handelt es sich um zwei verschiedene Polypropylene mit jeweils unterschiedlichem Copolymeranteil, die in einem Rohstoff zusammengefasst sind. Ein solches bimodales Polypropylen hat zwei Kristallitschmelzpunkte, wobei in der Regel über eine DSC-Analyse auch die ungefähren Anteile der zwei Polypropylene bestimmt werden können. Als Beispiel sei ein bimodales Polypropylen mit den Kristallitschmelzpunkten 125 °C und 143 °C mit einem Anteil der zwei verschiedenen Polypropylene von 25/75 genannt. Bei einer Erwärmungstemperatur von 130 °C wären erfindungsgemäß die 25 % Polypropylen mit Kristallitschmelzpunkt 125 °C der niedrig schmelzenden Polymerkomponente und die 75 % Polypropylen mit Kristallitschmelzpunkt 143 °C der hoch schmelzenden Polymerkomponente zuzurechnen.

In einer besonderen Ausführungsform wird eine Ausgangsfolienbahn mit folgenden Polymerbestandteilen verwendet: 25 bis 80 Gew.-%, insbesondere 25 bis 60 Gew.-% eines LLDPE, z. B. ein Ethylen-Octen-Copolymer mit 5 bis 15 Gew.-% Octenanteil; 20 bis 30 Gew.-% eines Propylen-Ethylen-Copolymeren mit 3 bis 12 Gew.-% Ethylen; und Rest LDPE; jeweils bezogen auf 100 Gew.-% niedrig und hoch schmelzende Polymerkomponente.

Ebenso wie sich in der niedrig schmelzenden Polymerkomponente spezielles geschmolzenes Polypropylen befinden kann, kann sich auch in der hoch schmelzenden Polymerkomponente ein spezielles nicht geschmolzenes Polyethylen befinden, das dann der hoch schmelzenden Polymerkomponente zugerechnet wird. Dies wird an folgendem Beispiel veranschaulicht. Eine für eine mikroporöse Ausgangsfolienbahn geeignete Rezeptur umfasst als Polymerbestandteile: 30 Gew.-% LDPE (Schmelzpunkt 112 °C), 30 Gew.-% LLDPE (Schmelzpunkt 124 °C), 20 Gew.-% HDPE (Schmelzpunkt 130 °C) und 20 Gew.-% Polypropylen (Schmelzpunkt 160 °C). Wird die Folienbahn auf eine Temperatur von 126 °C erwärmt, so befinden sich das LDPE und LLDPE erfindungsgemäß im schmelzeflüssigen Zustand, aber nicht nur das Polypropylen sondern auch das HDPE befinden sich nicht im schmelzeflüssigen Zustand.

Die mikroporösen Ausgangsfolienbahnen für die Durchführung des Verfahrens der Erfindung können durch beliebige, im Stand der Technik bekannte Verfahren hergestellt werden. Beispielsweise kann die Ausgangsfolienbahn hergestellt werden, indem die Polymerbestandteile und Füllstoffe in einem Extruder, z.B. einem Compounding-Extruder, auf eine Temperatur deutlich über der Schmelzflusstemperatur der Polymerbestandteile (z.B. über 200°C) erhitzt und verschmolzen werden. Dem schließt sich ein Castverfahren (Gießverfahren), z.B. über eine Schlitzdüse, oder ein Blasverfahren an. Diese Verfahren sind im Stand der Technik bekannt. Beim Schlitzdüsenverfahren wird eine Folie durch eine Breitschlitzdüse extrudiert. Bevorzugt ist das Blasverfahren, bei dem ein Blasschlauch gebildet wird. Die gebildete Schlauchfolie kann flach aufeinandergelegt und an den Enden aufgeschlitzt werden, so dass zwei Folienbahnen entstehen, von denen jede als Ausgangsfolienbahn verwendet werden kann.

Zur Erzeugung der Mikroporosität der Ausgangsfolienbahn kann die extrudierte Folie einem Reckvorgang unterzogen werden. Möglich ist ein Recken in Maschinenrichtung (MD), Querrichtung (CD), oder in Maschinen- und Querrichtung. Daneben ist auch ein Ringrolling möglich.

Die Ausgangfolienbahn kann gereckt sein. Ein Recken, Strecken oder Verstrecken einer Folie bedeutet eine Dehnung der Folie in eine angegebene Richtung, was zu einer Verringerung der Foliendicke führt. Die Folie kann in Maschinen- oder Längsrichtung (MD) gereckt sein, beispielsweise durch ein Reckwerk, das zwei oder mehrere Walzen umfasst, z.B. drei Walzen, die bei unterschiedlicher Geschwindigkeit angetrieben werden. Die Folie kann beispielsweise bei einem Reckverhältnis von 1:1,5 gereckt werden, was bedeutet, dass die Foliendicke von z.B. 15 µm auf 10 µm verringert wird. Es ist auch möglich, die Folienbahn zusätzlich einer Querverstreckung (CD) zu unterwerfen. Ein solches biaxiales Verstrecken kann beispielsweise durch auf dem Markt erhältliche Reckmaschinen, z.B. von der Firma Brückner, erreicht werden. Das verwendete Reckverhältnis hängt von der Folienrezeptur und den gewählten Verfahrensparametern ab und kann wenigstens 1:1,2, bevorzugt wenigstens 1:1,5, insbesondere wenigstens 1:2, stärker bevorzugt wenigstens 1:2,5, mehr bevorzugt wenigstens 1:3 oder wenigstens 1:4 betragen.

Die erfindungsgemäß verwendete Ausgangsfolienbahn enthält Füllstoffe. Geeignete Füllstoffe unterliegen keinen Beschränkungen und sind dem Fachmann bekannt. Es eignen sich alle Stoffe, die auf eine bestimmte Größe gemahlen werden können, im Extruder nicht schmelzen und nicht gereckt werden können. Besonders geeignet sind anorganische Füllstoffe, wie Kreide (Calciumcarbonat), Ton, Kaolin, Calciumsulfat (Gips) oder Magnesiumoxid. Daneben eignen sich auch synthetische Füllstoffe, wie Kohlenstofffasern, Cellulosederivate, gemahlene Kunststoffe oder Elastomere. Am meisten bevorzugt ist Calciumcarbonat oder Kreide wegen seines günstigen Preises, aber auch unter dem Gesichtspunkt der Nachhaltigkeit. Der Füllstoff kann eine Partikelgröße von z.B. 0,8 bis 2 µm haben. Falls ein Füllstoff mit einer gleichmäßigeren Partikelgröße als bei Kreide gewünscht ist, besteht auch die Möglichkeit, synthetische Füllstoffe mit einheitlicher Partikelgröße oder Partikelgrößenverteilung zu verwenden. Die Folie kann wenig Füllstoffe enthalten, z. B. 5 bis 45 Gew.-% oder 10 bis 50 Gew.-%, so dass sich beim Reckvorgang zwar Poren ausbilden, diese aber isoliert vorliegen und die Folie nicht atmungsaktiv ist. Um eine Atmungsaktivität der Folie zu erreichen, werden zweckmäßigerweise wenigstens 35 Gew.-% Füllstoff, insbesondere wenigstens 45 Gew.-% Füllstoff, bevorzugt wenigstens 55 Gew.-% Füllstoff, stärker bevorzugt wenigstens 65 Gew.-% Füllstoff, bezogen auf 100 Gew.-% der gesamten Rezeptur der Ausgangsfolienbahn, einschließlich Füllstoff(en), verwendet. Die Obergrenze an Füllstoff wird dadurch bestimmt, dass keine Poren mehr, sondern Löcher entstehen, oder die Folie reißt. Geeignete Folienrezepturen mit Füllstoff können vom Fachmann routinemäßig bestimmt werden. Besonders geeignet ist eine Rezeptur mit 35 bis 75 Gew.-%, insbesondere 45 bis 75 Gew.-%, bevorzugt 55 bis 70 Gew.-%, Füllstoff bezogen auf 100 Gew.-% Ausgangsfolienbahn. Beispielhafte Rezepturen für nicht-atmungsaktive Folien umfassen 5 bis 50 Gew.-%, insbesondere 10 bis 40 Gew.-%, Füllstoffe, bezogen auf 100 Gew.-% Ausgangsfolienbahn. Beispielhafte Rezepturen für atmungsaktive Folien umfassen 35 bis 80 Gew.-%, insbesondere 45 bis 75 Gew.-%, Füllstoffe, bezogen auf 100 Gew.-% Ausgangsfolienbahn. Es ist darauf zu achten, den Anteil der niedrig schmelzenden Komponente nicht so hoch zu wählen, dass eine Atmungsaktivität zwar erreicht wird, diese aber wieder verloren geht, weil die Poren sich wieder verschließen.

Ist die mikroporöse Ausgangsfolienbahn mehrschichtig und nicht atmungsaktiv, kann sich der Füllstoff in einer oder mehreren Folienlagen der Ausgangsbahn befinden. Ist die mikroporöse Ausgangsfolienbahn mehrschichtig und atmungsaktiv, kann sich der Füllstoff in einer oder mehreren Folienlagen der Ausgangsbahn befinden, wobei die Menge an Füllstoff in jeder Folienlage so gewählt wird, dass die Folienlage atmungsaktiv ist.

Die mikroporöse Ausgangsfolienbahn hat vorzugsweise Mikroporen in der Größe von Größe von 0,1 bis 5 µm, insbesondere von 0,1 bis 3 µm oder von 0,2 bis 1 µm. Daneben können auch einige wenige größere Poren vorliegen.

Die Ausgangsfolienbahn kann einschichtig oder mehrschichtig sein, also monoextrudiert bzw. coextrudiert sein. Es gibt keine Beschränkung bezüglich der Anzahl der verwendeten Schichten oder Lagen. Es können eine oder mehrere Schichten oder Lagen vorliegen, z. B. eine Schicht, zwei Schichten, drei Schichten oder vier Schichten. Möglich sind auch z. B. bis 5, 7 oder 9 Schichten. Die Schichten oder Lagen können gleiche oder unterschiedliche Rezepturen aufweisen, wobei die Zuordnung zur niedrig oder hoch schmelzenden Polymerkomponente jeweils durch den Kristallitschmelzpunkt bestimmt wird. Die Schichten oder Lagen können durch Blasextrusion oder Castextrusion hergestellt werden. Bei mehrschichtigen Ausgangsfolienbahnen kann wenigstens eine Lage durch Blasextrusion und wenigstens eine andere Lage durch Castextrusion hergestellt werden. Es besteht keine Beschränkung bei der Kombination von blasextrudierten und/oder castextrudierten Folien, Folienschichten oder Folienlagen. Es besteht keine Beschränkung bei der Anzahl der coextrudierten Schichten oder Lagen.

In anderen Ausführungsformen ist die Ausgangsfolienbahn nicht coextrudiert.

Die Ausgangsfolienbahn kann in beispielhaften Ausführungsformen auch wie nachstehend beschrieben hergestellt werden:
- blasextrudiert, geschlitzt, auf zwei separate Bahnen bzw. separate Rollen;
- blasextrudiert, geschlitzt, auf zwei oder mehrere separate Bahnen gleichzeitig;
- blasextrudiert, geschlitzt, flachgelegt als nicht getrennter Schlauch;
- blasextrudiert, geschlitzt in zwei oder mehrere separate Bahnen, die von verschiedenen Extrudern kommen; oder
- castextrudiert in zwei oder mehrere separate Bahnen gleichzeitig.

Es ist auch möglich, die Folien Inline herzustellen. In diesem Fall liegt ein Produktionsschritt für die Prozesse Extrusion und Recken (MDO, biaxial oder Ringrolling) sowie die weitere Verarbeitung (z. B. Prägen und Bedruckung) vor.

Die beim Verfahren der Erfindung eingesetzten Ausgangsfolienbahnen können eingefärbt sein, z. B. weiß mit Titandioxid. Weiterhin können die Ausgangsfolienbahnen übliche Zusatzstoffe und Verarbeitungshilfsmittel enthalten. Insbesondere handelt es sich hierbei neben den bereits genannten Füllstoffen um Pigmente oder andere farbgebende Stoffe, Antihaftmittel, Gleitmittel, Verarbeitungshilfsmittel, antistatische Mittel, keimverhindernde Mittel (Biozide), Antioxidationsmittel, Wärmestabilisierungsmittel, Stabilisierungsmittel gegenüber UV-Licht oder andere Mittel zur Eigenschaftsmodifizierung. Typischerweise werden solche Stoffe, ebenso wie Füllstoffe, bereits vor dem erfindungsgemäßen Erwärmen der Ausgangsfolienbahn zugegeben, z.B. bei deren Herstellung in die Polymerschmelze oder vor dem Extrudieren zu einer Folie.

Die Ausgangsfolienbahn weist bevorzugt Flächengewichte im Bereich unter 50 g/m², insbesondere unter 40 g/m², bevorzugt unter 30 g/m², stärker bevorzugt unter 20 g/m², auf. Möglich sind auch Flächengewichte im Bereich unter 10 g/m² oder unter 5 g/m². Bevorzugte Flächengewichte liegen im Bereich von 1 bis 30 g/m², 1 bis 25 g/m² oder 1 bis 20 g/m², insbesondere von 1 bis 15 g/m², stärker bevorzugt von 2 bis 10 g/m² oder 7 bis 20 g/m². Die Flächengewichte können auch 1 bis 10 g/m², 5 bis 10 g/m² oder 5 bis 15 g/m² betragen. Die Ausgangsfolienbahn kann Dicken im Bereich von 2 bis 30 µm, insbesondere von 2 bis 15 µm, 5 bis 20 µm oder 5 bis 10 µm aufweisen.

Bei dem Verfahren der Erfindung wird die Erwärmung der Ausgangsfolienbahn bis zum oder oberhalb dem schmelzeflüssigen Zustand der niedrig schmelzenden Polymerkomponente und unterhalb des schmelzeflüssigen Zustands der hoch schmelzenden Polymerkomponente durchgeführt. Bis zum schmelzeflüssigen Zustand bedeutet hierbei, dass sich die niedrig schmelzende Polymerkomponente im schmelzeflüssigen Zustand befindet. Es wird jedoch nur so hoch erwärmt, dass sich die hoch schmelzende Polymerkomponente nicht im schmelzeflüssigen Zustand befindet.

Um eine stabile Prozessführung auch über einen längeren Zeitraum zu ermöglichen, sollten zweckmäßigerweise die (Kristallit-)Schmelzpunkte der niedrig und hoch schmelzenden Polymerkomponenten nicht zu nahe beieinander liegen. Vorzugsweise liegt der Kristallitschmelzpunkt der niedrig schmelzenden Polymerkomponente oder, bei Anwesenheit mehrerer niedrig schmelzender Polymerkomponenten, der Kristallitschmelzpunkt derjenigen mit dem höchsten Kristallitschmelzpunkt davon, mindestens etwa 5 °C, verzugsweise mindestens etwa 10 °C und insbesondere mindestens etwa 20 °C unterhalb des Kristallitschmelzpunkts oder des schmelzeflüssigen Zustandes der hoch schmelzenden Polymerkomponente oder, bei Anwesenheit mehrerer hoch schmelzender Polymerkomponenten, derjenigen mit dem niedrigsten Kristallitschmelzpunkt davon.

Zur Erreichung des schmelzeflüssigen Zustands der niedrig schmelzenden Polymerkomponente, jedoch nicht des schmelzeflüssigen Zustands der hoch schmelzenden Polymerkomponente, unterliegt der speziell gewählte Temperaturabstand keinen besonderen Beschränkungen, sofern die vorgenannte Bedingung erfüllt ist. Der gewählte Temperaturabstand wird zweckmäßigerweise durch praktische Überlegungen bezüglich Sicherheit der Verfahrensdurchführung oder durch wirtschaftliche Überlegungen bestimmt. Wenn z.B. bei einer bestimmten Temperatur die niedrig schmelzende Polymerkomponente aufgeschmolzen ist, bringt eine weitere Temperaturerhöhung keine besseren Ergebnisse. Zudem steigt der Wärmeverbrauch und man kommt gegebenenfalls zu nahe an den Schmelzbereich der hoch schmelzenden Polymerkomponente heran, so dass das Verfahren schwieriger durchzuführen ist. Vorzugsweise wird deshalb das Verfahren der Erfindung so durchgeführt, dass die Erwärmung der Ausgangsfolienbahn auf 5 bis 20 °C, bevorzugt 5 bis 15 °C oder 10 bis 20°C, insbesondere 10 bis 15°C oder 15 bis 20 °C, unterhalb des Kristallitschmelzpunkts der hoch schmelzenden Polymerkomponente erfolgt. Alternativ wird die Erwärmung insbesondere bei einer Temperatur im Bereich von 1 bis 20 °C, bevorzugt 2 bis 10°C, oberhalb des Kristallitschmelzpunkts oder schmelzeflüssigen Zustands der niedrig schmelzenden Polymerkomponente(n) durchgeführt. Es ist sicherzustellen, dass die Kristallitschmelzpunkte der niedrig schmelzenden Polymerkomponente(n) erreicht werden.

Erfindungsgemäß kann die Erwärmung der Ausgangsfolienbahn mittels wenigstens einer Heizwalze erfolgen. Vorzugsweise erfolgt die Erwärmung mittels einer oder mehrerer Heizwalzen, bei denen es sich um Kontaktwalzen handelt, die mittels eines Wärmeträgers, z.B. Dampf, Wasser, Öl, auf die vorgegebene Temperatur erhitzt werden. In einer bevorzugten Ausführungsform wird eine einzelne Heiz- oder Kontaktwalze eingesetzt. Es ist aber auch möglich, zwei oder mehrere Heizwalzen einzusetzen, wobei sichergestellt werden muss, dass der schmelzeflüssige Zustand der niedrig schmelzenden Polymerkomponente vor dem Kühlwalzenspalt erreicht wird. Um zu gewährleisten, dass die Ausgangsfolienbahn die Temperatur der Heizwalze tatsächlich erreicht oder dass bei hohen Produktionsgeschwindigkeiten (bei denen die Oberflächentemperatur des Heizzylinders höher im Vergleich zur Folie ist) der schmelzeflüssige Zustand der niedrig schmelzenden Polymerkomponente sicher erreicht wird, ist für eine ausreichende Verweilzeit der Ausgangsfolienbahn auf der Heizwalzenoberfläche zu sorgen. Dies kann über eine entsprechende Umschlingungsstrecke des Heizzylinders, den Heizwalzendurchmesser und/oder die Folienbahngeschwindigkeit nach Maßgabe der Foliendicke erfolgen. Zweckmäßigerweise kann eine Heizwalze mit antihaft-beschichteter Oberfläche verwendet werden, um ein leichteres Ablösen der Folienbahn von der Heizwalze zu erreichen und damit ein Abreißen der Folienbahn zu verhindern. Dadurch wird eine Verschiebung des Ablösepunktes in Drehrichtung der Heizwalze vermieden und es ist keine oder nur mehr eine geringe Voreilung nötig. Beispielsweise wird hierzu eine PTFE (Polytetrafluorethylen)-beschichtete Heizwalze verwendet.

Die Erwärmung der Folienbahn kann auch mit anderen Erwärmungsmethoden, wie Strahlungswärme, z.B. mit Infrarotheizung oder -strahlern, durchgeführt werden. Es kann auch zusätzlich zu einer oder mehreren Heizwalzen eine andere Erwärmung, z.B. Infrarotheizung, vorgesehen sein.

In einer bevorzugten Ausführungsform wird die gefüllte, gereckte Folienbahn in Abwesenheit einer Vliesbahn bis zum teilweise schmelzflüssigen Zustand erwärmt. Anders als in der EP 1 784 306 A1 kann vorliegend ohne eine Vliesbahn zwischen dem Heizzylinder und der Folienbahn in den teilweise schmelzflüssigen Zustand erwärmt werden. In bevorzugten Ausführungsformen erfolgt die Erwärmung der mikroporösen Ausgangsfolienbahn über einen Heizzylinder, wobei zwischen dem Heizzylinder und der Folienbahn kein Vlies vorliegt. In diesen Ausführungsformen steht die Folienbahn in direktem Kontakt mit dem Heizzylinder. Möglich ist auch, dass zwischen dem Heizzylinder und der Folienbahn kein Vlies vorliegt, und eine Vliesbahn auf der Folienbahn aufliegt oder darüber geführt wird. In anderen bevorzugten Ausführungsformen wird die Erwärmung der mikroporösen Ausgangsfolienbahn über einen Heizzylinder in Abwesenheit eines Vlieses durchgeführt. Das bedeutet, es liegt kein Vlies zwischen dem Heizzylinder und der Folienbahn vor und es liegt kein Vlies auf der Folienbahn auf. In alternativen Ausführungsformen liegt ein Vlies zwischen dem Heizzylinder und der Folienbahn vor.

Erfindungsgemäß wird die Folienbahn nach der Erwärmung durch einen gekühlten Walzenspalt geführt. Die den Kühlwalzenspalt bildenden Walzen sind so gekühlt, dass eine schnelle und schlagartige Abkühlung erreicht wird. Eine Abkühlung auf eine Temperatur unterhalb des Kristallitschmelzpunktes der niedrig schmelzenden Polymerkomponente, vorzugsweise auf wenigstens 5 °C °C darunter, insbesondere auf wenigstens 10 °C darunter, ist zweckmäßig. Bevorzugte Abkühlbereiche sind 5 bis 10 °C, stärker bevorzugt 10 bis 30 °C, unterhalb des Kristallitschmelzpunktes der niedrig schmelzenden Polymerkomponente. Beispielsweise kann die Kühlung der Walzen mit Wasser in einem Temperaturbereich von 5 bis 20 °C, z.B. Wasser von etwa 10 °C, erfolgen. Der Abstand zwischen der Heizwalze oder, bei Verwendung mehrerer Heizwalzen, der letzten Heizwalze und/oder anderer Erwärmungsquellen und dem Kühlwalzenspalt wird hierbei wegen möglicher Wärmeverluste nicht zu groß gewählt.

Bei dem Kühlwalzenspalt kann es sich im einfachsten Fall z.B. um einen Glattwalzenspalt mit zwei glatten Walzen handeln. Im Fall von Hygienefolien wird der Walzenspalt jedoch vorzugsweise von einem Walzenpaar mit einer Strukturwalze und einer Glattwalze (z. B. Gummiwalze) gebildet, wodurch die Folienbahn eine strukturierte Oberfläche erhält. Bevorzugte Strukturen im Hygienebereich sind Mikrostrukturen, z.B. ein Pyramidenstumpf. Vorzugsweise besteht der gekühlte Walzenspalt aus einer Stahlwalze und einer im Gegendruck arbeitenden Gummiwalze, wobei die Stahlwalze mit der strukturierten Oberfläche versehen ist. Die Stahlwalze kann mit einer textilähnlichen Gravur ausgestattet sein, die das textile Erscheinungsbild der Oberfläche der Folie verstärkt. Eine Prägestruktur der Stahlwalze reduziert weiterhin des Glanzgrad der Folie.

Die Geschwindigkeit der den Kühlwalzenspalt bildenden Walzen kann so gewählt werden, dass diese gleich der Geschwindigkeit der Heizwalze oder, bei Verwendung mehrerer Heizwalzen, der letzten Heizwalze ist, so dass die Folie dazwischen nicht gereckt wird. Die Geschwindigkeit der den Kühlwalzenspalt bildenden Walzen kann auch so gewählt werden, dass diese größer oder kleiner als die Geschwindigkeit der Heizwalze oder, bei Verwendung mehrerer Heizwalzen, der letzten Heizwalze ist, so dass die Folie dazwischen gereckt oder geschrumpft wird. Wegen Wärmeverlusten sollte der Abstand zwischen Heizwalze und Kühlwalzenspalt möglichst gering gehalten werden.

In Abhängigkeit von den Folienparametern und anderen Verfahrensbedingungen bewegen sich die Folienbahngeschwindigkeiten im Bereich von 50 bis 900 m/min. Die Geschwindigkeit der Heizwalze(n) beträgt vorzugsweise 50 bis 900 m/min, insbesondere 50 bis 800 m/min, bevorzugt 100 bis 600 m/min. Die Geschwindigkeit der den Kühlwalzenspalt bildenden Walzen beträgt vorzugsweise 50 bis 900 m/min, insbesondere 50 bis 800 m/min, bevorzugt 100 bis 600 m/min. Die Geschwindigkeiten der Heizwalze(n) und Kühlwalzen werden so gewählt, dass in Abhängigkeit von der Folienrezeptur und den gewählten Verfahrensparametern diese gleich sind oder aber verschieden sind, so dass die Folie im gewünschten Verhältnis gereckt oder geschrumpft (Annealing) wird. Das erfindungsgemäße Verfahren ermöglicht die Herstellung von Folien mit sehr geringen Flächengewichten von z. B. 2, 3, 4, 5, 6, 7, 8, 9, 10, 11, 12, 13, 14, 15, 16, 17, 18, 19, 20 oder 25 g/m².Die entsprechenden Foliendicken liegen im Bereich von z. B. 25, 20, 19, 18, 17, 16, 15, 14, 13, 12, 11, 10, 9, 8, 7, 6 oder auch nur 5 µm. Bevorzugte Folien weisen eine Dicke im Bereich von 2 bis 13 µm oder 4 bis 25 µm auf oder haben ein Flächengewicht von 1 bis 15 g/m² oder von 4 bis 25 g/m² oder von 7 bis 20 g/m².

Die erfindungsgemäß erhaltenen Folien verfügen, trotzdem sie sehr dünn und mikroporös sind, über ausgezeichnete mechanische Eigenschaften und über zusätzlich noch immer hohe Durchstoßfestigkeiten (d.h. Widerstand gegenüber Superabsorberkörnern z.B. in Windeln) und hohe Thermostabilitäten (d.h. Widerstand gegenüber Hotmelt-Klebern).

Erfindungsgemäß erhaltene Folien können in bekannter Weise weiterverarbeitet werden. Beispielsweise können Single-Backsheets oder Vlies-Folien-Laminaten daraus hergestellt werden. Zur Herstellung von Vlies-Folien-Laminaten können die Folien mit Vliesen mittels Klebstoffen, bevorzugt inline, verklebt werden. Daneben können Vlies-Folien-Laminate auch durch dem Fachmann bekanntes Thermobonding hergestellt werden, bei dem das Material von einer erfindungsgemäß erhaltenen Folie und/oder von Vlies punktuell mittels zweier beheizter Walzen, meist einer Prägewalze (gravierte Stahlwalze) und einer glatten Stahlwalze als Gegenwalze, durch hohe Temperatur und Druck angeschmolzen wird und dadurch die Folie und das Vlies miteinander verbunden werden. Darüber hinaus können Vlies-Folien-Laminate wie oben beschrieben auch mittels Thermolaminierung hergestellt werden. Eine Thermolaminierung ist insbesondere bei sehr dünnen Folien, z.B. unter 10 g/m² oder z. B. 4 g/m² bevorzugt. Daneben können Vlies-Folien-Laminate auch mittels Ultraschall-Laminierung (z. B. mit Ultraschall Herrmann Technologie) hergestellt werden. Die hergestellten Vlies-Folien-Laminate können in an sich bekannter Weise weiterverarbeitet werden, wobei auch ein Recken in Maschinen- oder Querrichtung oder in beide Richtungen möglich ist. Ebenso können Single-Backsheets weiterverarbeitet werden.

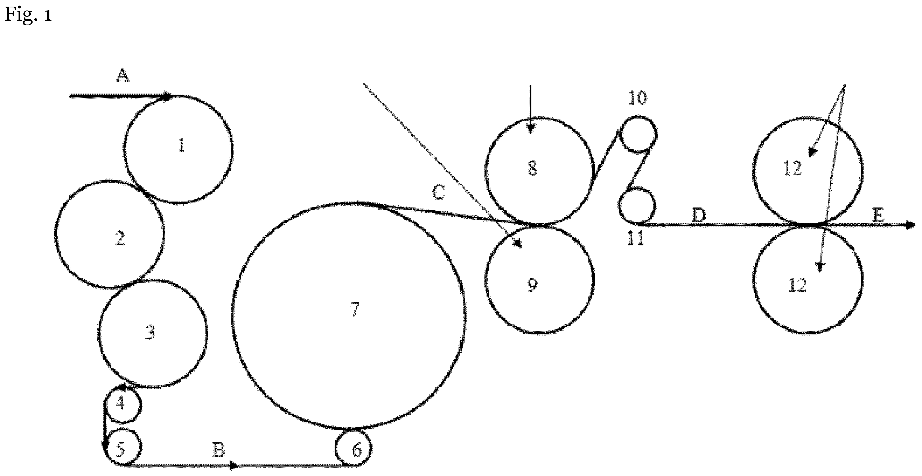

Figur 1 zeigt eine bevorzugte Ausführungsform zur Durchführung des erfindungsgemäßen Verfahrens. Eine gefüllte Folie A, z.B., aus dem Extruder, läuft über ein Reckwerk mit den Walzen 1, 2 und 3, wodurch die Folie mikroporös wird. Die drei Walzen können beispielsweise bei Walzengeschwindigkeiten von 100/200/200 m/min oder 100/150/200 m/min betrieben werden. Über die Umlenkwalzen 4, 5 und 6 läuft die mikroporöse Ausgangsfolienbahn B auf eine Heizwalze 7. Bei der Heizwalze 7 handelt es sich z.B. um eine antihaftend beschichtete Stahlwalze, die mittels Wärmezufuhr auf die gewünschte Oberflächentemperatur erhitzt wird. Die Folienbahn läuft dann auf der Heizwalze 7 und wird dabei erfindungsgemäß in den teilweise schmelzeflüssigen Zustand erwärmt. Von der Heizwalze 7 läuft die teilweise schmelzeflüssige Folienbahn C in einen Kühlwalzenspalt, der von den Walzen 8 und 9 gebildet wird. Die Walze 8 ist vorzugsweise als Struktur- oder Prägewalze ausgebildet, wodurch die Folienbahn eine Prägestruktur oder strukturierte Oberfläche erhält. Die Walze 9 ist vorzugsweise eine Gummiwalze. Das Walzenpaar 8/9 ist vorzugsweise wassergekühlt, z.B. mit Wasser von etwa 10 °C. Die den Kühlspalt bildenden Walzen 8 und 9 werden so angetrieben, dass gegenüber der Bahngeschwindigkeit der Heizwalze 7 eine höhere, niedrigere oder gleiche Geschwindigkeit besteht. Im Kühlwalzenspalt wird die Folienbahn schockartig gekühlt und geprägt. Nach dem Walzenpaar 8/9 kann die Folie direkt abgenommen werden, oder über Umlenkwalzen 10 und 11, die auch gekühlt werden können, kann die Folienbahn D beispielsweise einem Druckwerk mit den Walzen 12 zugeführt werden, um sie zu bedrucken. Danach kann die Folie E abgenommen werden.

Die Erfindung ermöglicht aufgrund der Herstellung von Folien äußerst geringer Dicken eine Einsparung an Rohstoffen und trägt somit zur Ressourcenschonung und Nachhaltigkeit bei. Sie leistet somit einen Beitrag zur Umweltschonung. Dies gilt für Folien auf dem Hygienesektor wie auch für andere Anwendungen, vor allem in den Anwendungen, in denen die Folien in starkem Maße als Bestandteile von Wegwerfprodukten verwendet werden.

Die erfindungsgemäß hergestellte Folie bietet im Hinblick auf die eingangs beschriebenen Probleme im Stand der Technik demgegenüber zahlreiche Verbesserungen und Vorteile:
- Die Folie erlaubt eine hohe Temperaturbelastung, z. B. bei Hotmeltklebern.
- Die Folie zeigt fast keinen Nachschrumpf.
- Die Folie kann auch aufgrund ihrer Prägestruktur Blocking verhindern, da diese Struktur wie ein Abstandshalter zwischen den Folienlagen wirkt und damit ein Aneinanderkleben der Folieneinzellagen verhindert.
- Die Folie kann auch aufgrund ihrer Prägestruktur einerseits das Aneinanderkleben der Folieneinzellagen verhindern und andererseits die Planlage der Folie erhöhen, da die Prägestruktur Dick- und Dünnstellen ausgleicht. Eine eventuelle Randverdickung durch Neck In kann über die Einstellung des Prägedrucks egalisiert werden.
- Die Folie hat eine erhöhte Weiterreißfestigkeit (wegen "Weichglühen" der Folie nach dem Reckprozess). Die dreidimensionale Prägestruktur verhindert das leichte Einreißen der Folie, da durch die Prägestruktur eine Folienausrichtung in z-Richtung vorhanden ist.
- Die Folie hat eine erhöhte Durchstoßfestigkeit. Die dreidimensionale Prägestruktur verhindert ein punktuelles Einreißen der Folie über die Superabsorberkörner, da eine Folienausrichtung in z-Richtung durch die Prägestruktur vorhanden ist.
- Zu groß gewordene Poren können schrumpfen und damit können hohe Liquid Impact Werte reduziert werden. Das bedeutet, dass zu groß gewordene Poren von > 1µm durch das erfindungsgemäße Verfahren auf eine Größe von <1µm geschrumpft werden können. Im Prägewerk wird über den Anstelldruck und die schockartige Kühlung eine Prägestruktur in die Folie eingebracht und zugleich die Porengröße eingefroren und damit fixiert.
- Da die Folien kaum messbaren Nachschrumpf zeigen, können sie inline direkt nach dem Reckvorgang bedruckt werden, beispielweise mit einem zwischengeschalteten "Heißprägeverfahren".
- Die Folie zeigt bei großen thermischen Belastungen, wie beispielsweise beim Auftrag von Hotmelt-Klebersystemen, höhere Widerstandskräfte bzw. geringes Schrumpfverhalten bzw. geringere sog. Burn Through-Effekte, wodurch die Lochbildung verringert wird bzw. nicht mehr auftritt.

Die erfindungsgemäß erhaltenen Folien können in vielen Bereichen verwendet werden. Sie finden Verwendung im Hygiene- oder Medikalbereich, z.B. als Wäscheschutzfolie oder allgemein als flüssigkeitsundurchlässige Sperrschicht, insbesondere als Backsheets in Windeln, Damenbinden, Betteinlagen oder in ähnlichen Produkten. Weiterhin können die Folien in anderen technischen Gebieten Anwendung finden, wie im Bausektor als Baufolien, z. B. für Dachunterspannbahnen, Estrichabdeckungen oder Wandabdeckungen, oder als Automobilbereich als Autoschutzfolien.

Erfindungsgemäß erhaltene Folien können in bekannter Weise weiterverarbeitet werden, beispielsweise zu Vlies-Folien-Laminaten. Zur Herstellung solcher Laminate können sie mit Klebstoffen, bevorzugt inline, verklebt werden. Daneben können Vlies-Folien-Laminate auch durch dem Fachmann bekanntes Thermobonding hergestellt werden, bei dem das Material von einer erfindungsgemäß erhaltenen Folie und/oder von Vlies punktuell mittels zweier beheizter Walzen, meist einer Prägewalze (gravierte Stahlwalze) und einer glatten Stahlwalze als Gegenwalze, durch hohe Temperatur und Druck angeschmolzen wird und dadurch die Folie und das Vlies miteinander verbunden werden. Darüber hinaus können Vlies-Folien-Laminate auch mittels Thermolaminierung, beispielsweise wie in EP 1 784 306 B1 beschrieben, hergestellt werden. Eine Thermolaminierung ist insbesondere bei sehr dünnen Folien, z.B. 4 g/m² bevorzugt. Alternativ können Vlies-Folien-Laminate auch mittels Ultraschall-Laminierung (z. B. mit Ultraschall Herrmann Technologie) hergestellt werden. Die hergestellten Vlies-Folien-Laminate können in an sich bekannter Weise weiterverarbeitet werden.

Die Erfindung wird anhand des nachfolgenden Beispiels näher erläutert, ohne die Erfindung zu beschränken.

### BEISPIELE

Die Beispiele veranschaulichen die erfindungsgemäße Herstellung von Folien und zeigen ihr Verhalten beim Warmschrumpf.

### Beispiel 1

Die Folien werden hergestellt, indem eine kreidegefüllte Folie aus zwei verschiedenen Polypropylen-Compounds gemäß üblichen Verfahren blasextrudiert wird. Die beiden Polypropylen-Compounds weisen einen unterschiedlichen Schmelzpunkt/-bereich auf. Das höherschmelzende Polypropylen-Compound enthält 92,5 Gew.-% %Polypropylen (PP) und 7,5 Gew.-% Ethylen-Copolymer. Es hat einen DSC-Schmelzpunkt von etwa 164°C und wird in einer Menge von 16 Gew.-% eingesetzt, bezogen auf 100 Gew.-% des eingesetzten Polymermaterials (d.h. ohne Füllstoff). Das niedrig schmelzende Polypropylen-Compound enthält 90,5 Gew.-% %PP und 9,5 Gew.-% Ethylen-Copolymer. Es hat einen DSC-Schmelzpunkt von etwa 138°C und wird in einer Menge von 80 Gew.-% eingesetzt, bezogen auf 100 Gew.-% des eingesetzten Polymermaterials. Jedes Compound hat einen Kreideanteil von etwa 60% (bezogen auf 100 Gew.-% Compound). Es werden noch 4 Gew.-% eines LDPE (DSC-Schmelzpunkt 111 °C), bezogen auf 100 Gew.-% des eingesetzten Polymermaterials zugegeben.

Die gefüllte, extrudierte Folie wird nach dem Extrusionsprozess einer Reck-Präge-Druckmaschine gemäß Figur 1 zugeführt. Dabei wird die Folie A mittels der temperierten Reckwalzen 1/2/3 in Maschinenrichtung ca. 2:1 verstreckt, um die Atmungsaktivität zu erzeugen. Anschließend wird die atmungsaktive Folie B über den Heizzylinder 7 bei verschiedenen Heizzylindertemperaturen, wie in Tabelle 1 unten angegeben, erwärmt. Nach der Erwärmung der Folie über dem Heizzylinder 7 wird die Folie C unter Beibehaltung dieses Zustandes dem Prägewerk (Prägewalze 8 und Gummiwalze 9) zugeführt und darin mit einer Prägestruktur versehen. Die Präge-Gummiwalzen sind stark gekühlt (<30°C) und befinden sich im gegenseitigen Anstelldruck von mehreren Tonnen, beispielsweise bei Werten von 4 bis 10 Tonnen Anstelldruck auf jeder Walzenseite. Die Folie D verlässt schockartig gekühlt und geprägt das Prägewerk.

Die Tabelle 1 gibt an, wie sich eine Folie in Abhängigkeit von der verwendeten Heizzylindertemperatur beim Warmschrumpf verhält.

Der Warmschrumpf wurde wie folgt gemäß ASTM D2732-96 bestimmt.

### Beschreibung

Bei der Überprüfung des Warmschrumpfes nach ASTM D2732-96 wird der Grad der Materialorientierung bestimmt.

Hierbei wird eine definierte Probe (10 x 10 cm) in einem Wasserbad bei 80°C erhitzt, und nach 30 Sekunden wieder abgekühlt.

Als Schrumpfwert wird die Längendifferenz der Probe in % angegeben.

### Prüfmittel

Schrumpfbad, Maßband

### Probenvorbereitung

Auf den Proben wird ein Quadrat von 100 x 100 mm mittels Blechquadrat aufgezeichnet,
und mit einem Maßband kontrolliert.

Die Proben sind mit Längs- bzw. Querrichtung zu kennzeichnen.

### Durchführung

Schrumpfbad auf 80° Celsius einstellen.

Proben in das erhitzte Wasserbad eintauchen, nach 30 Sekunden vorsichtig entnehmen und abkühlen lassen (Wasserbad im Spülbecken).

### Auswertung

Schrumpfwerte längs und quer: der Unterschied zwischen der ursprünglich markierten Messlänge und der geschrumpften Messlänge entspricht dem Schrumpfwert in %.

Die Messergebnisse gemäß Tabelle 1 wurden bei den angegebenen Heizzylindertemperaturen erhalten. Die angegebenen Heizzylindertemperaturen beziehen sich auf eine Messung innen am Heizzylinder über einen Temperatursensor (Platin-Messwiderstand, Pt100 Element). Die in Tabelle 1 angegebene Schrumpfrichtung gibt an, in welcher Richtung (Maschinenrichtung md oder Querrichtung cd) die Schrumpfung der Folie gemessen wurde.

**Tabelle 1**

| Heizzylindertemperatur [°C] | Flächengewicht [g/m²] | Schrumpfrichtung | Schrumpf im Wasserbad bei 80°C [%] |
|---|---|---|---|
| 140 | 19,4 | md | 1,6 |
| | | cd | 0,3 |
| 137 | 18,8 | md | 2,5 |
| | | cd | 0,7 |
| 135 | 19,4 | md | 2,5 |
| | | cd | 0,7 |
| 133 | 19,0 | md | 3,9 |
| | | cd | 0,7 |
| 131 | 19,0 | md | 4,4 |
| | | cd | 0,5 |
| 129 | 19,4 | md | 5,3 |
| | | cd | 0,6 |
| 127 | 19,7 | md | 7,5 |
| | | cd | 0,2 |
| 125 | 19,7 | md | 7,5 |
| | | cd | 0,3 |
| 123 | 19,4 | md | 8,3 |
| | | cd | 0,2 |
| 121 | 20,0 | md | 9,6 |
| | | cd | 0,2 |
| Ohne Heizzylinder & Kühlung (Prägung) | 22,0 | md | 24,1 |
| | | cd | 0,2 |

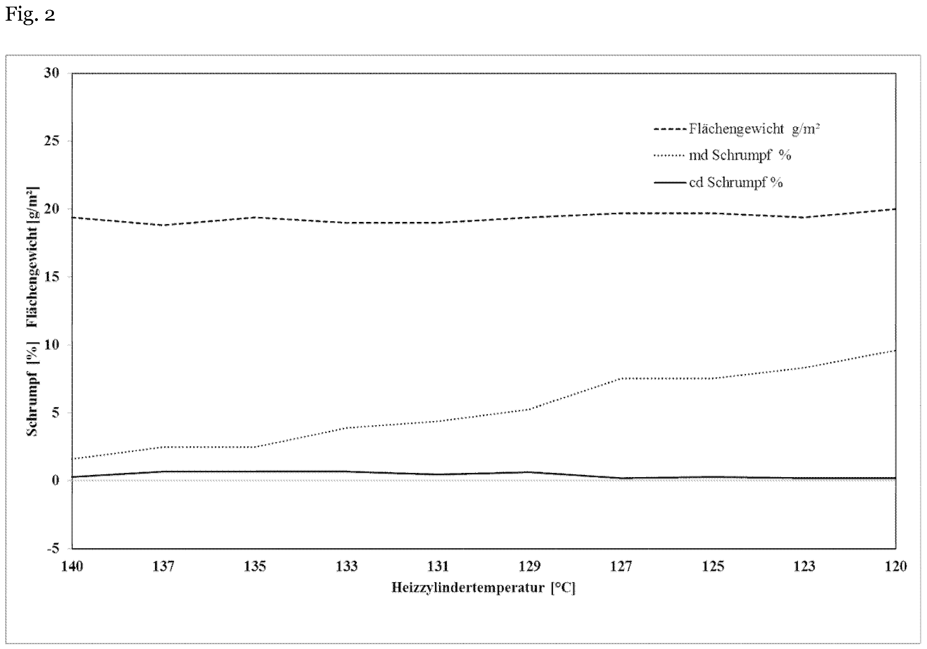

Tabelle 1 zeigt deutlich, wie sich der Nachschrumpf verringert, wenn die Folie bei ansteigender Heizzylindertemperatur in den teilweise schmelzflüssigen Zustand geführt wird. Die Ergebnisse sind auch in Figur 2 als Diagramm graphisch dargestellt. Das Diagramm zeigt, in Abhängigkeit von der Heizzylindertemperatur, zum einen das Flächengewicht der Folie (oberer Teil des Diagramms; gestrichelte Linie) und zum anderen den Warmschrumpf (in einem Wasserbad von 80°C) in md-Richtung (punktierte Linie) und cd-Richtung (durchgezogene Linie). Der Wert für den in Tabelle 1 angegebenen Schrumpf bei Arbeiten ohne Heizzylinder und Kühlung (Prägung) ist in Fig. 2 nicht gezeigt. Der Wert in Tabelle 1 zeigt deutlich, wie hoch der Schrumpf ohne Durchführung des erfindungsgemäßen Verfahrens ist.

### Beispiel 2

Eine Folie wurde gemäß dem in Beispiel 1 beschriebenen Verfahren bei einer Heizzylindertemperatur von 135 °C mit einem Flächengewicht von 16 g/m² hergestellt. Diese Folie wurde mit einer Folie aus dem Stand der Technik (Vergleichsfolie) mit einem Flächengewicht von 22 g/m² im Hinblick auf den Warmschrumpf bei verschiedenen Wasserbadtemperaturen, wie in Tabelle 2 angegeben, verglichen. Die Schrumpfmessung wurde analog zu Beispiel 1 durchgeführt. Die Ergebnisse sind in Tabelle 2 gezeigt.

**Tabelle 2**

| Wasserbadtemperatur [°C] | Schrumpfrichtung | Schrumpf [%] | |
|---|---|---|---|
| | | Folie [16g/m²] | Vergleich [22g/m²] |
| <40 | md | 0 | 0 |
| | cd | 0 | 0 |
| 40 | md | 0 | 1,1 |
| | cd | 0 | 0 |
| 50 | md | 0 | 2,2 |
| | cd | 0 | 0 |
| 60 | md | 0,6 | 3,8 |
| | cd | 0 | 0 |
| 70 | md | 1,2 | 9,1 |
| | cd | 0,7 | 0 |
| 75 | md | 2,0 | 11,3 |
| | cd | 0,3 | -0,5 |
| 80 | md | 2,0 | 13,1 |
| | cd | 0-0,2 | -1,6 |

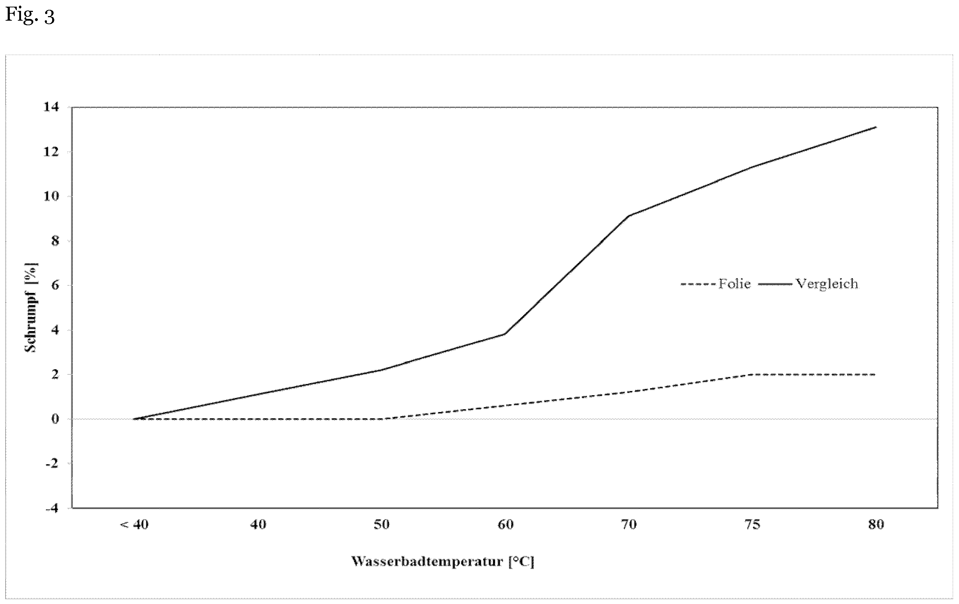

Die Ergebnisse von Tabelle 2 sind für die md-Schrumpfrichtung auch in Figur 3 graphisch dargestellt. So zeigt das Diagramm von Figur 3 den Schrumpf in md-Richtung für die erfindungsgemäße Folie (gestrichelte Linie) und die Vergleichsfolie (Vergleich; durchgezogene Linie) in Abhängigkeit von der angegebenen Wasserbadtemperatur.

Aus Tabelle 2 lässt sich entnehmen, dass die erfindungsgemäße Folie einen weitaus geringeren Warmschrumpf zeigt als das Vergleichsprodukt. Die erfindungsgemäße Folie bietet deshalb große Vorteile bei hohen Temperaturen, wie sie z. B. in Lagerräumen oder Schiffen auftreten können. Sie würde bei großen thermischen Belastungen, wie beispielsweise beim Auftrag von Hotmelt-Klebersystemen, höhere Widerstandskräfte bzw. geringes Schrumpfverhalten bzw. geringere sog. Burn Through-Effekte zeigen.

## Patentansprüche

1. Verfahren zur Herstellung einer gefüllten Folienbahn aus einer mikroporösen Ausgangsfolienbahn aus thermoplastischem Polymermaterial, die wenigstens eine niedrig schmelzende Polymerkomponente, eine hoch schmelzende Polymerkomponente und einen Füllstoff enthält, wobei das Verfahren folgende Schritte umfasst:
Erwärmen der mikroporösen Ausgangsfolienbahn bis zum teilweise geschmolzenen Zustand, bei dem sich wenigstens eine niedrig schmelzende Polymerkomponente im schmelzeflüssigen Zustand und wenigstens eine hoch schmelzende Polymerkomponente nicht im schmelzeflüssigen Zustand befindet, und
Abkühlen durch Führen der teilweise geschmolzenen Folienbahn durch einen gekühlten Walzenspalt.

2. Verfahren nach Anspruch 1, **dadurch gekennzeichnet, dass** die mikroporöse Ausgangsfolienbahn einlagig ist.

3. Verfahren nach Anspruch 1, **dadurch gekennzeichnet, dass** die mikroporöse Ausgangsfolienbahn mehrlagig ist, insbesondere dass die mikroporöse Ausgangsfolienbahn eine coextrudierte Folienbahn ist.

4. Verfahren nach einem der Ansprüche 1 bis 3, **dadurch gekennzeichnet, dass** die mikroporöse Ausgangsfolienbahn gereckt worden ist, insbesondere dass die Ausgangsfolienbahn nach ihrer Extrusion bei einem Reckverhältnis von 1,2:1 bis 4:1, bevorzugt 1,5:1 bis 3:1, gereckt worden ist.

5. Verfahren nach einem der vorhergehenden Ansprüche, **dadurch gekennzeichnet, dass** die mikroporöse Ausgangsfolienbahn 10 bis 90 Gew.-% Füllstoff, insbesondere 20 bis 80 Gew.-% Füllstoff, insbesondere 30 bis 75 Gew.-% Füllstoff, bezogen auf 100 Gew.-% der Ausgangsfolienbahn, enthält.

6. Verfahren nach einem der vorhergehenden Ansprüche, **dadurch gekennzeichnet, dass** die mikroporöse Ausgangsfolienbahn atmungsaktiv ist.

7. Verfahren nach einem der vorhergehenden Ansprüche, **dadurch gekennzeichnet, dass** die mikroporöse Ausgangsfolienbahn eine Wasserdampfdurchlässigkeit im Bereich von 500 bis 5000 g/m² in 24h bei Messung gemäß ASTM E398 bei 37,8 °C und 90 % relativer Feuchtigkeit aufweist.

8. Verfahren nach einem der vorhergehenden Ansprüche, **dadurch gekennzeichnet, dass** die mikroporöse Ausgangsfolienbahn 15 bis 85 Gew.-% niedrig schmelzende Polymerkomponente und 85 bis 15 Gew.-% hoch schmelzende Polymerkomponente, bezogen auf 100 Gew.-% niedrig und hoch schmelzende Polymerkomponente, umfasst.

9. Verfahren nach einem der vorhergehenden Ansprüche, **dadurch gekennzeichnet, dass** eine Ausgangsfolienbahn mit wenigstens einem Polyethylen als niedrig schmelzender Polymerkomponente und mit wenigstens einem Polypropylen als hoch schmelzender Polymerkomponente verwendet wird.

10. Verfahren nach einem der vorhergehenden Ansprüche, **dadurch gekennzeichnet, dass** die Erwärmung der mikroporösen Ausgangsfolienbahn über einen Heizzylinder erfolgt, wobei zwischen dem Heizzylinder und der Folienbahn kein Vlies vorliegt.

11. Verfahren nach einem der vorhergehenden Ansprüche, **dadurch gekennzeichnet, dass** die mikroporöse Ausgangsfolienbahn durch Blasextrusion, Castextrusion, oder Blasextrusion und Castextrusion hergestellt worden ist.

12. Verfahren nach einem der vorhergehenden Ansprüche, **dadurch gekennzeichnet, dass** die Folienbahn im gekühlten Walzenspalt einer Abkühlung auf wenigstens 10 bis 30 °C unterhalb des Kristallitschmelzpunktes wenigstens einer niedrig schmelzenden Polymerkomponente unterworfen wird.

13. Folienbahn, erhältlich durch ein Verfahren nach einem der vorhergehenden Ansprüche, wobei die mikroporöse Ausgangsfolienbahn eine Wasserdampfdurchlässigkeit im Bereich von 500 bis 5000 g/m² in 24h bei Messung gemäß ASTM E398 bei 37,8 °C und 90 % relativer Feuchtigkeit aufweist.

14. Folienbahn nach Anspruch 13, mit einem Flächengewicht im Bereich von 1 bis 30 g/m², insbesondere von 5 bis 25 g/m², bevorzugt von 7 bis 20 g/m², stärker bevorzugt von 10 bis 20 g/m².

15. Verwendung der Folienbahn nach Anspruch 13 oder 14 im Hygiene- oder Medikalbereich, insbesondere für Backsheets in Windeln, für Betteinlagen oder Damenbinden.

16. Verwendung der Folienbahn nach Anspruch 13 oder 14 im Baubereich oder als Automobilschutzfolie.

## Claims

1. A process for producing a filled film web from a microporous starting film web of thermoplastic polymer material, which comprises at least one low-melting polymer component, one high-melting polymer component and a filler, the process comprising the following steps:
heating of the microporous starting film web to a partly molten state in which at least one low-melting polymer component exists in a molten liquid state and at least one high-melting polymer component does not exist in the molten liquid state, and
cooling down by passing the partly molten film web through a cooled roller nip.

2. The process according to claim 1, **characterized in that** the microporous starting film web is single-layered.

3. The process according to claim 1, **characterized in that** the microporous starting film web is multi-layered, in particular **in that** the microporous starting film web is a co-extruded film web.

4. The process according to any of claims 1 to 3, **characterized in that** the microporous starting film web has been stretched, in particular **in that** the starting film web after its extrusion has been stretched at a stretching ratio of 1.2:1 to 4:1, preferably 1.5:1 to 3:1.

5. The process according to any of the preceding claims, **characterized in that** the microporous starting film web contains 10 to 90 % by weight of filler, in particular 20 to 80 % by weight of filler, in particular 30 to 75 % by weight of filler, based on 100 % by weight of the starting film web.

6. The process according to any of the preceding claims, **characterized in that** the microporous starting film web is breathable.

7. The process according to any of the preceding claims, **characterized in that** the microporous starting film web has a water vapor permeability within the range of from 500 to 5000 g/m² in 24 hours when measured according to ASTM E398 at 37.8 °C und 90 % relative humidity.

8. The process according to any of the preceding claims, **characterized in that** the microporous starting film web comprises 15 to 85 % by weight of low-melting polymer component and 85 to 15 % by weight of high-melting polymer component, based on 100 % by weight of low-melting and high-melting polymer components.

9. The process according to any of the preceding claims, **characterized in that** a starting film web with at least one polyethylene serving as a low-melting polymer component and with at least one polypropylene serving as a high-melting polymer component is used.

10. The process according to any of the preceding claims, **characterized in that** the heating of the microporous starting film web is performed by means of a heating cylinder, wherein a non-woven is not present between the heating cylinder and the film web.

11. The process according to any of the preceding claims, **characterized in that** the microporous starting film web has been produced by blown film extrusion, cast extrusion, or by blown film extrusion and cast extrusion.

12. The process according to any of the preceding claims, **characterized in that** the film web is subjected to cooling in the cooled roller nip to at least 10 to 30°C below the crystallite melting point of at least one low-melting polymer component.

13. A film web obtainable by a process according to any of the preceding claims, wherein the microporous starting film web has a water vapor permeability within the range of from 500 to 5000 g/m² in 24 hours when measured according to ASTM E398 at 37.8 °C und 90 % relative humidity.

14. The film web according to claim 13, having a basis weight within the range of from 1 to 30 g/m², in particular from 5 to 25 g/m², preferably from 7 to 20 g/m², more preferable from 10 to 20 g/m².

15. Use of the film web according to claim 13 or 14 in the hygiene or medical field, in particular for back sheets of diapers, for mattress protectors or sanitary napkins.

16. Use of the film web according to claim 13 or 14 in the construction area or as automobile protection film.

## Revendications

1. Procédé de fabrication d'une feuille continue chargée à partir d'une feuille continue de départ microporeuse en matériau polymère thermoplastique, laquelle contient au moins un composant polymère à bas point de fusion, un composant polymère à haut point de fusion et une charge, le procédé comprenant les étapes suivantes :
chauffage de la feuille continue de départ microporeuse jusqu'à l'état partiellement fondu dans lequel au moins un composant polymère à bas point de fusion se trouve à l'état liquéfié et au moins un composant polymère à haut point de fusion ne se trouve pas à l'état liquéfié, et
refroidissement par passage de la feuille continue partiellement fondue à travers un intervalle refroidi entre des rouleaux.

2. Procédé selon la revendication 1, **caractérisé en ce que** la feuille continue de départ microporeuse est monocouche.

3. Procédé selon la revendication 1, **caractérisé en ce que** la feuille continue de départ microporeuse est multicouche, en particulier **en ce que** la feuille continue de départ microporeuse est une feuille continue coextrudée.

4. Procédé selon une des revendications 1 à 3, **caractérisé en ce que** la feuille continue de départ microporeuse a été étirée, en particulier **en ce qu'**après son extrusion la feuille continue de départ a été étirée selon un rapport d'étirage de 1,2:1 à 4:1, préférentiellement de 1,5:1 à 3:1.

5. Procédé selon une des revendications précédentes, **caractérisé en ce que** la feuille continue de départ microporeuse contient 10 à 90 % en poids de charge, en particulier 20 à 80 % en poids de charge, en particulier de 30 à 75 % en poids de charge, rapportés à 100 % en poids de la feuille continue de départ.

6. Procédé selon une des revendications précédentes, **caractérisé en ce que** la feuille continue de départ microporeuse est respirante.

7. Procédé selon une des revendications précédentes, **caractérisé en ce que** la feuille continue de départ microporeuse présente une perméabilité à la vapeur d'eau située dans la plage de 500 à 5 000 g/m² en 24 heures mesurée selon ASTM E398 à 37,8 °C et à 90 % d'humidité relative.

8. Procédé selon une des revendications précédentes, **caractérisé en ce que** la feuille continue de départ microporeuse comprend 15 à 85 % en poids de composant polymère à bas point de fusion et 85 à 15 % en poids de composant polymère à haut point de fusion, rapportés à 100 % en poids de composant polymère à bas et haut point de fusion.

9. Procédé selon une des revendications précédentes, **caractérisé en ce qu'**une feuille continue de départ avec au moins un polyéthylène comme composant polymère à bas point de fusion et avec au moins un polypropylène comme composant polymère à haut point de fusion est utilisée.

10. Procédé selon une des revendications précédentes, **caractérisé en ce que** le chauffage de la feuille continue de départ microporeuse s'effectue par l'intermédiaire d'un cylindre de chauffage, entre le cylindre de chauffage et la feuille continue ne se trouvant aucun non-tissé.

11. Procédé selon une des revendications précédentes, **caractérisé en ce que** la feuille continue de départ microporeuse a été fabriquée par extrusion soufflage, moulage par extrusion ou par extrusion soufflage et moulage par extrusion.

12. Procédé selon une des revendications précédentes, **caractérisé en ce que**, dans l'intervalle refroidi entre des rouleaux, la feuille continue est soumise à un refroidissement à au moins 10 à 30 °C en dessous du point de fusion des cristallites au moins d'un composant polymère à bas point de fusion.

13. Feuille continue pouvant être obtenue par l'intermédiaire d'un procédé selon une des revendications précédentes, la feuille continue de départ microporeuse présente une perméabilité à la vapeur d'eau située dans la plage de 500 à 5 000 g/m² en 24 heures mesurée selon ASTM E398 à 37,8 °C et à 90 % d'humidité relative.

14. Feuille continue selon la revendication 13, avec un poids surfacique dans la plage de 1 à 30 g/m², en particulier de 5 à 25 g/m², préférentiellement de 7 à 20 g/m², plus préférentiellement de 10 à 20 g/m².

15. Utilisation de la feuille continue selon la revendication 13 ou 14 dans le domaine de l'hygiène ou du médical, en particulier pour des feuilles de fond dans des couches, pour des alèses ou des serviettes périodiques.

16. Utilisation de la feuille continue selon la revendication 13 ou 14 dans le domaine de la construction ou comme feuille de protection en automobile.
